# EUROPÄISCHE PATENTANMELDUNG

(11) **EP 1 359 178 A1**
(43) Veröffentlichungstag der Anmeldung: **05.11.2003**
(21) Anmeldenummer: 03008669.8
(22) Anmeldetag: 16.04.2003
(51) Int. Cl.: C08G 64/30, C07F 9/54

(54) **Verfahren zur Herstellung einer flüssigen Formulierung von Phosphoniumphenolaten**

(30) Priorität: 29.04.2002 DE 10219027
(71) Anmelder: Bayer Aktiengesellschaft, 51368 Leverkusen (DE)
(72) Erfinder: Möthrath, Melanie, Dr., 40227 Düsseldorf (DE); Bunzel, Lothar, 47906 Kempen (DE); Kratschmer, Silke, Dr., 60488 Frankfurt (DE); Rechner, Johann Dr., 47906 Kempen (DE)

(57) **Zusammenfassung**

Die Anmeldung betrifft ein Verfahren zur Herstellung einer flüssigen Formulierung von Phosphoniumphenolaten und die Verwendung der erfindungsgemäß hergestellten flüssigen Formulierung von Phosphoniumphenolaten als Umesterungskatalysatoren, insbesondere für die lösungsmittelfreie Herstellung von therrnoplastischen Polycarbonaten.

## Beschreibung

Die Anmeldung betrifft ein Verfahren zur Herstellung einer flüssigen Formulierung von Phosphoniumphenolaten und die Verwendung der erfindungsgemäß hergestellten flüssigen Formulierung von Phosphoniumphenolaten als Umesterungskatalysatoren, insbesondere für die lösungsmittelfreie Herstellung von thermoplastischen Polycarbonaten.

Die Herstellung von Phosphoniumphenolaten ist aus DE-A-197 273 51 und WO 01/46100 A1 bekannt. Darin werden Verfahren zur Synthese hochreiner Phosphoniumphenolate, die als Phenoladdukte in kristalliner Form anfallen, beschrieben. Die so erhaltenen Phenoladdukte der Phosphoniumphenolate werden großtechnisch, nach Auflösen bei Temperaturen oberhalb von 45°C in Phenol, als Umesterungskatalysatoren in Schmelzeumesterungsverfahren zur Herstellung von Polycarbonat eingesetzt.

Für den Einsatz der Phenoladdukte von Phosphoniumphenolaten als Umesterungskatalysatoren im Schmelzeumesterungsverfahren spielt die Reinheit der Katalysatoren eine entscheidende Rolle, da Verunreinigungen zu Aktivitätsschwankungen, Verfärbungen oder Nebenreaktionen im Umesterungsprozess führen können.

Aus DE-A-199 107 45 ist eine bei Raumtemperatur flüssige Formulierung von Tetraphenylphosphoniumphenolat (TPP-P) mit Phenol bei einer Zusammensetzung von 28 bis 45 Gew.% TPP-P in Phenol bekannt. Dies ermöglicht eine Katalysatorzugabe im flüssigen Zustand ohne thermische Vorbelastung und Fremd- und Zusatzstoffe. Die Herstellung der flüssigen Formulierung erfolgt über das Abmischen der nach DE-A-197 273 51 und WO 01/4600 A1 erhältlichen hochreinen, kristallinen Phenoladdukte von TPP-P mit entsprechenden Mengen Phenol, um die Reinheit der entstehenden Formulierung zu gewährleisten. Großtechnisch ist jedoch eine direkte Dosierung der so erhältlichen, bei Raumtemperatur flüssigen, Katalyator-Formulierung im kontinuierlichen Schmelzeumesterungsverfahren zur Herstellung von Polycarbonat aufgrund der hohen Katalysator-Konzentrationen unpraktikabel. Eine weitere Verdünnung mit Phenol ist notwendig, wodurch diese Verfahrensweise insgesamt äußerst umständlich wird. Weiterhin erweist es sich im großen Maßstab als schwierig, bei den verschiedenen Mischungs- und Verdünnungsschritten inerte Bedingungen einzuhalten, die eine konstante Qualität des Katalysators gewährleisten.

Wenn aber eine unkomplizierte direkte Synthese von den in DE 198 10 745 A1 beschriebenen, bei Raumtemperatur flüssigen Katalysatorformulierungen von TPP-P möglich wäre, würden diese Formulierungen Vorteile bei der Abfüllung und Lagerung bieten. So ist in der Regel die Herstellung der Phenoladdukte der Phosphoniumphenolate apparatetechnisch bzw. physikalisch nicht mit dem Umesterungsprozess verbunden. Das macht eine Lagerung des Katalysators bis zum Einsatz notwendig. Um dabei eine konstant hohe Qualität des Katalysators zu gewährleisten, ist deshalb eine einfache, möglichst viele äußere Einflüsse unterbindende und inerte Katalysatorabfüllung in inerte, lichtundurchlässige Gebinde nach der Herstellung des Katalysators wünschenswert. Hier würde eine flüssige Katalysatorformulierung Vorteile bieten. Gleichzeitig sollte der Katalysator beim Lagern eine möglichst geringe Oberfläche aufweisen und unkompliziert unter inerten Bedingungen dem Schmelzeprozess zugeführt werden können. Weiterhin wäre es im Hinblick auf die Herstellung von hochreinem Polycarbonat für optische Anwendungen nach dem Schmelzeumesterungsverfahren von Vorteil, wenn man den Katalysator durch Filtration von Kleinstpartikeln befreien kann. Auch diese Probleme sind mit einer flüssigen Katalysatorformulierung gut zu lösen.

Aufgabe der vorliegenden Anmeldung ist somit ein Verfahren zu finden, mit dem eine einfache direkte Herstellung von einer bei Raumtemperatur flüssigen Formulierung von Tetraphenylphosphoniumphenolat mit Phenol gelingt und die Reinheit des Phosphoniumphenolats möglichst hoch ist.

Überraschenderweise wurde nun gefunden, dass durch die Umsetzung von Phosphoniumhalogeniden und Phenolen in wässrig-alkalischer Lösung eine direkte Herstellung von bei Raumtemperatur flüssigen, hochreinen Formulierungen von Tetraphenylphosphoniumphenolat mit Phenol möglich ist.

Gegenstand der vorliegenden Erfindung ist somit ein Verfahren zur Direktherstellung von bei Raumtemperatur flüssigen, hochreinen Formulierungen von Tetraphenylphosphoniumphenolat mit Phenol aus Tetraphenylphosphoniumhalogeniden und Phenol in wässrig-alkalischer Lösung bei Temperaturen von 0 bis 55°C, das dadurch gekennzeichnet ist, dass man die Umsetzung im Molverhältnis Phenol zu Tetraphenylphosphoniumhalogenid von ≥ 10:1und bei pH-Werten von 9,5 bis 11 durchführt und nach der Umsetzung schwerlösliche Alkohole in Gewichtsverhältnissen von wässriger Reaktionslösung zu Alkohol zwischen 2:1 und 1:2 zusetzt, wobei die Alkohole eine Löslichkeit in reinem Wasser von < 20 Gew.%, bevorzugt < 15 Gew.% haben.

Die Umsetzung wird vorzugsweise bei Temperaturen von 0 bis 55°C, insbesondere 15 bis 50°C durchgeführt.

Die Umsetzung wird vorzugsweise bei Molverhältnissen von Phenol zu Phosphoniumhalogenid von ≥ 10 :1, besonders bevorzugt bei 10:1 bis 13:1 durchgeführt.

Die Umsetzung wird vorzugsweise bei pH-Werten von 9.5 bis 11 durchgeführt.

Nach der Umsetzung werden schwerlösliche Alkohole in Mengen von 50 Gew.-% bis 200 Gew.-%, vorzugsweise von 55 Gew.-% bis 140 Gew.-%, bezogen auf Gewichtsmenge der wässrigen Reaktionslösung zugegeben, wobei die Alkohole vorzugsweise eine Löslichkeit in reinem Wasser von < 20 Gew.%, bevorzugt < 15 Gew.% haben.

Die derart hergestellte flüssige Formulierung von Tetraphenylphosphoniumphenolat enthält nicht mehr als 0.3 Gew.-%, bevorzugt 0,1 Gew.-% Halogenid.

Die derart hergestellte flüssige Formulierung von Tetraphenylphosphoniumphenolat enthält nicht mehr als 1 ppm, bevorzugt 0,5 ppm alkalischen Natriumverbindungen.

Für die Umsetzung werden insbesondere Tetraphenylphosphoniumhalogenide der Formel (I) 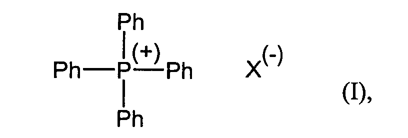 worin
- X⁽⁻⁾: für ein Halogenidion, vorzugsweise F⁽⁻⁾, Cl⁽⁻⁾ oder Br⁽⁻⁾ steht,
eingesetzt.

Bevorzugt beschreibt Formel (I) Tetraphenylphosphoniumbromid.

Derartige Tetraphenylphosphoniumhalogenide und ihre Herstellung sind bekannt oder nach bekannten Methoden erhältlich (siehe beispielsweise "Houben-Weyl, Methoden der organischen Chemie" Band XII/1, Seiten 79 ff. und Worrall, J. Amer. Chem. Soc. 52 (1930), Seiten 293 ff.).

Diese Verbindungen (I) entstehen bei der Umsetzung von Triphenylphosphin mit Halogenarylen oder Halogenalkylen, z.B. Benzylbromid in Gegenwart von Metallsalzen (Friedel-Crafts-Alkylierung) oder in Gegenwart von Grignard-Verbindungen und Kobalt(II)-chlorid.

Für die Umsetzung bevorzugte Phenole sind Phenol oder substitutierte Phenole sowie Bisphenole.

Besonders bevorzugte Phenole sind die der Formel (II) 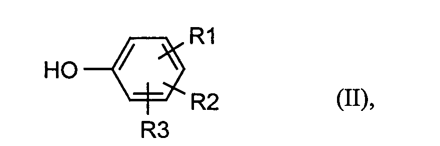 worin
- R₁ bis R₃: unabhängig voneinander für H, C₁-C₁₂-Alkyl, C₅-C₆-Cycloalkyl, C₇-C₁₂-Arylalkyl und C₆-C₁₄-Aryl stehen; vorzugsweise stehen R₁ bis R₃ für Wasserstoff.

Derartige Phenole sind literaturbekannt.

Bevorzugt werden Tetraphenylphosphoniumphenolate der Formel (III) hergestellt 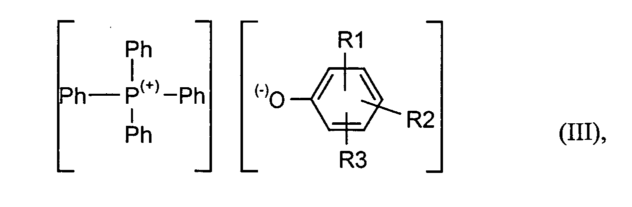 worin die Reste R₁ bis R₃ die oben angegebenen Bedeutungen haben.
Für die Herstellung der wässrig-alkalischen Phase wird bevorzugt VE-Wasser bzw. destilliertes Wasser verwendet.

Der pH-Wert von 9.5 bis 11.0 wird vorzugsweise mit Hilfe einer Alkalilauge, vorzugsweise Natronlauge oder Kalilauge - unter Berücksichtigung der puffernden Wirkung von Phenol/Na-Phenolat - eingestellt.

Das erfindungsgemäße Verfahren kann kontinuierlich oder diskontinuierlich durchgeführt werden, wobei eine diskontinuierliche Arbeitsweise bevorzugt wird.

Nach einer bevorzugten Arbeitsweise werden Phenol, Tetraphenylphosphoniumhalogenid und Wasser als Lösung bei 40°C vorgelegt. Gegebenenfalls unter Kühlung wird der pH-Wert unter Zugabe von Alkalilauge auf Werte von 9.5 bis 11.0 eingestellt. Dabei wird vorzugsweise unter intensiver Mischung der Reaktionskomponenten die Temperatur bei 0 bis 55°C, vorzugsweise bei 10 bis 50°C gehalten. Die Reaktionsdauer sollte unter 2 Stunden, vorzugsweise unter 1 Stunde liegen.

Nach der Umsetzung wird der wässrigen Reaktionslösung soviel eines in Wasserschwerlöslichen Alkohols zugesetzt, dass sich die wässrige Phase von der organischen Phase trennt und letztere mindestens 1 mal, vorzugsweise 5 mal mit VE-Wasser bzw. destilliertem Wasser extrahiert wird. Unter schwerlöslich sind dabei solche Alkohole zu verstehen, deren Löslichkeit in Wasser < 20 Gew.%, bevorzugt < 15 Gew.% ist.
Anschließend wird die abgetrennte organische Phase durch Destillation im Vakuum vom Alkohol befreit, so dass man eine farblose bei Raumtemperatur flüssige Formulierung von Tetraphenylphosphoniumphenolaten zurück behält.

Erfindungsgemäß geeignete Alkohole für die Reaktionslösung sind aliphatische Alkohole der Formel CₙH₂ₙ₊₁-OH, worin n eine ganze Zahl von 4 bis 10 einschließlich ist, wie z.B. n-Propanol, Isopropanol, n-Butanol, Isobutanol, n-Pentanol, Methylbutanole, Neopentanol, Amylalkohole, verzweigte und unverzweigte Hexanole, Heptanole, Octanole, Nonanole oder Decanole.

Erfindungsgemäß geeignete Alkohole für die Reaktionslösung sind auch cycloaliphatische Alkohole der Formel CₙH₂ₙ₋₁-OH, worin n eine ganze Zahl von 5 bis 10 einschließlich ist, wie z.B. Cyclopentanol, Methylcyclopentanole, Cyclopentanmethanol, Cyclopentylpropanole, Cyclohexanol, Cyclohexylethanole, Cyclohexylpropanole, Cyclohexylbutanole, Methyl-, Ethyl-, Propyl- und Butylhexanole, Cycloheptanole, Cyclooctanole.

Erfindungsgemäß können auch mehrwertige aliphatische oder cycloaliphatische Alkohole eingesetzt werden.

Bevorzugte aliphatische Alkohole sind Propanole, (Iso)Butanole, Pentanole und Hexanole, insbesondere Isobutanol und Isopropanol.

Bevorzugte cycloaliphatische Alkohole sind Cyclopentanol, Cycloheptanol und Cyclooctanol, besonders bevorzugt Cyclohexanol.

Nach der Zugabe des Alkohols, nach abgeschlossener Umsetzung zum Phosphoniumphenolat, liegt das Gewichtsverhältnis von Wasser zu Alkohol liegt zwischen 2:1 und 1:2, vorzugsweise zwischen 1:1 und 1:2.

Die erfindungsgemäß einzusetzenden Alkohole werden zur besseren Aufarbeitung zugesetzt, da die Mischung Phenol/Alkohol eine geringere Dichte als die wässrige Lösung hat und somit die organische über der wässrigen Phase ist. Somit kann die wässrige Phase unten abgelassen werden, die organische Phase, die das Phenolat enthält, kann dann im selben Trenngefäß mit VE-Wasser gewaschen werden und das Waschwasser wiederum unten abgelassen werden.

Setzt man den Alkohol nicht zu, dann ist nur die salzhaltige wässrige Lösung schwerer als die organische Phase und kann unten abgelassen werden. Bei der weiteren Wäsche mit VE-Wasser kommt es zur Phasenumkehr, die organische Phase ist schwerer und daher unter der wässrigen Phase. Diese Aufarbeitung erweist sich dahingehend als aufwendiger, dass ein zweites Aufarbeitungsgefäß notwendig ist.

Erfindungsgemäß hergestellte quartäre Phosphoniumphenolate sind insbesondere die Verbindungen der Formeln

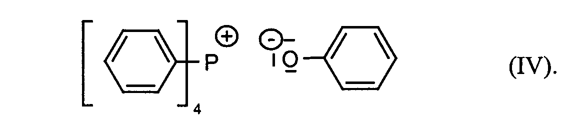

Mit dem erfindungsgemäßen Verfahren gelingt es, eine bei Raumtemperatur flüssige Formulierung von Tetraphenylphosphoniumphenolat in hohen Ausbeuten und hoher Reinheit herzustellen.

Vorzugsweise wird die so hergestellte bei Raumtemperatur flüssige Formulierung von Tetraphenylphosphoniumphenolat oder anderen Phosphoniumphenolaten nach der Isolierung unter inerten Bedingungen in ein inertes, lichtundurchlässiges Lagergebinde abgefüllt, mit Stickstoff oder anderen inerten Gasen beschleiert und verschlossen.

Vorzugsweise wird die bei Raumtemperatur flüssige Formulierung von Tetraphenylphosphoniumphenolat flüssig in geschlossenen Gebinden bei Temperaturen von 0 bis 40°C, bevorzugt 5 bis 35°C bis zu einem Zeitraum von 5 Jahren, bevorzugt 3 Jahren gelagert.

Die so hergestellte und gelagerte flüssige Formulierung von Tetraphenylphosphoniumphenolat eignet sich besonders als Katalysator zur Veresterung und zur Umesterung, insbesondere zur Herstellung von Polycarbonaten nach dem Schmelzumesterungsverfahren (siehe US-A 3 442 854).

Das Schmelzumesterungsverfahren geht bekanntlich beispielsweise von aromatischen Diphenolen, Kohlensäurediarylestern und gegebenenfalls Verzweigern und/oder Monophenolen aus.

Die erfindungsgemäß erhältliche flüssige Formulierung von Tetraphenylphosphoniumphenolat wird hierbei als Katalysator in Mengen von 10⁻¹ mol bis 10⁻⁸ mol, vorzugsweise in Mengen von 10⁻³ mol bis 10⁻⁷ mol, pro mol Diphenol eingesetzt.

In einer alternativen Verfahrensweise wird die erfindungsgemäß erhältliche flüssige Formulierung von Tetraphenylphosphoniumphenolat vor dem Einsatz als Katalysator im Schmelzeumesterungsverfahren filtriert, vorzugsweise erfolgt die Filtration in zwei Stufen. Der Filter für die zweite Stufe besitzt eine absolute Porengröße von 3 µ, bevorzugt 1 µ am meisten bevorzugt 0,5 µ.

Weitere Einzelheiten des Schmelzeumesterungsverfahrens sind in der Literatur beschrieben (siehe beispielsweise Hermann Schnell, Chemistry and Physics of Polycarbonates, Polymer Reviews, Vol. 9, 1964, Seiten 44 bis 51, DE-A 1 031 512, US-A 3 022 272, US-A 5 340 905 und US-A 5 399 659).

Die mit der erfindungsgemäß erhältlichen flüssigen Formulierung von Tetraphenylphosphoniumphenolat hergestellten thermoplastischen Polycarbonate sind lösungsmittelfrei, mit heller Eigenfarbe ausgestattet und weitgehend frei von unerwünschten Fehlstellen im Polycarbonat.

Der technische Einsatz dieser so hergestellten Polycarbonate in Form der verschiedensten Formteile ist überall dort möglich, wo bislang bereits thermoplastische Polycarbonate eingesetzt wurden, etwa in der Elektrotechnik, als Lampenabdeckungen, als Sicherheitsscheiben oder als optische Datenspeicher, z. B. CD-Material.

### Beispiele

Die folgenden Beispiele sollen die Erfindung illustrieren, ohne sie jedoch einzuschränken.

Zur Identifikation von Tetraphenylphosphoniumphenolat wird ein ³¹P-NMR-Spektrum aufgenommen. Die Substanz wird dazu in Deuterochloroform gelöst und vermessen.

Der Gehalt an Tetraphenylphosphoniumkationen und Phenol/Phenolat wird mittels ¹³C-NMR bestimmt. Dazu wird ein Spektrum aufgenommen, die Signale von Tetraphenylphosphoniumkationen und Phenol/Phenolat werden integriert und auf ihre Molprozente normiert auf 100 % angegeben.

Der Gehalt an Phosphor wird so bestimmt, dass eine abgewogene Menge der Probe mit Salpetersäure erhitzt und danach in Schwefel- und Perchlorsäure aufgelöst wird. Die entstehende Phosphorsäure wird mit Ammoniummolybdat präzipitiert. Das Präzipitat wird in einem Filtertiegel gesammelt, und durch Wiegen bestimmt man den Phosphorgehalt.

Der Gehalt an Brom wird so bestimmt, dass eine abgewogene Menge der Substanz in einem Wickbold Apparat verbrannt wird und die Verbrennungsgase absorbiert werden. Von dieser Lösung wird der Bromid-Gehalt mittels lonen-Austausch-Chromatographie ermittelt.

Der Natrium-Gehalt wird über Atomabsorptionsspektroskopie und ICP ermittelt.

Der Wassergehalt nach Karl-Fischer wird nach ISO 760 (Determination of Water - Karl Fischer Method, 1st ed., 1978-12-01) bestimmt.

Die Farbzahl wurde als Differenz der Extinktion bei 420 nm und 700 nm in Dichlormethan bei einer Konzentration von 2,4 g/50 ml und einer Schichtdicke von 10 cm ermittelt.

Die relative Lösungsviskosität wurde in Dichlormethan bei einer Konzentration von 5 g/l bei 25°C bestimmt.

Der Gehalt an phenolischem OH wird durch IR-Messung erhalten. Zu diesem Zweck wird eine Differenzmessung von einer Lösung aus 2 g Polymer in 50 ml Dichlormethan gegenüber reinem Dichlormethan vermessen und die Extinktionsdifferenz bei 3582 cm⁻¹ bestimmt.

### Vergleichsbeispiel 1

Nach WO 01/46100 und DE 198 10 745 A1 wird eine bei Raumtemperatur flüssige Formulierung von Tetraphenylphosphoniumphenolat hergestellt.

In einem 2-1-Rundkolben mit Rührer, Thermometer und Tropftrichter werden 376 g (4,0 mol) Phenol, 800 ml 3E-Wasser, 335,44 g (0,8 mol) Tetraphenylphosphoniumbromid und 640 g Isobutanol vorgelegt und bei 20°C bis 25°C gerührt. Innerhalb von ca. 5 Minuten werden 79 g (0,97 mol) 49 %ige Natronlauge zugetropft, der pH-Wert wird auf einen Bereich von 9,5 bis 11,0 eingestellt. Anschließend wird 0,5 h bei 45°C gerührt. Nach der Phasentrennung wird die untere wässrige Phase abgelassen und die organische Phase dreimal mit VE-Wasser gewaschen, das Waschwasser als schwerere Phase wird jeweils unten abgelassen. Anschließend wird die organische Phase unter Rühren auf Raumtemperatur abgekühlt. Dabei kristallisiert das Produkt aus. Nach mindestens 4 h Kristallisationszeit wird das Produkt abgesaugt. Das Filtrat wird nach NMR-Analyse auf den Gehalt an Phenol, Isobutanol und Tetraphenylphosphoniumphenolat wieder der Reaktion zugeführt. Der kristalline Rückstand wird mit 2-Propanol nachgewaschen, dann bei 100°C im Wasserstrahlvakuum getrocknet.

Man erhält ein kristallines Produkt mit einem TPP-P-Gehalt von 64,4 Gew.% und einem Phenol-Gehalt von 35,5 Gew.%. Der Wassergehalt liegt unter 0,05 %, der Brom-Gehalt unter 0,01 % und der Natrium-Gehalt unter 0,5 ppm. Der Phosphor-Gehalt ist 4,6 %.

30 g Kristalle TPP-P*2PhOH nehmen ein Volumen von 52 ml ein.

Ein Teil des so hergestellten kristallinen TPP-P*2PhOH wird mit Phenol im molaren Verhältnis 40 zu 60 unter N₂-Atmosphäre in 20 ml Rollrandfläschchen mit Magnetrührer eingewogen (Gesamtmenge 10 g) und mit einem Septum verschlossen. Diese Fläschchen werden in eine automatische Schütteleinrichtung gespannt und in ein temperiertes Wasserbad getaucht. Die Temperatur des Wasserbades wird nun schrittweise (5°C/h) solange erhöht, bis die Mischung flüssig vorliegt, gleichzeitig sorgt die Schütteleinrichtung für eine optimale Vermischung. Nach dem Aufschmelzen wird die Probe aus dem Wasserbad entnommen und auf Raumtemperatur abgekühlt. Die Probe bleibt flüssig; der Stockpunkt liegt bei -3°C.

### Beispiel 1

In einem 2-1-Rundkolben mit Rührer, Thermometer und Tropftrichter werden 209 g (2,22 mol) Phenol, 79 ml VE-Wasser und 83,86 g (0,2 mol) Tetraphenylphosphoniumbromid vorgelegt und bei 40°C gerührt. Innerhalb von ca. 5 Minuten werden 25,64 g (0,30 mol) 47 %ige Natronlauge zugetropft, der pH-Wert wird auf einen Bereich von 9,5 bis 11,0 eingestellt. Anschließend wird 1 h bei 50°C gerührt. Dann werden 160 g Isobutanol zugegeben. Nach der Phasentrennung wird die untere wässrige Phase abgelassen und die organische Phase fünfmal mit VE-Wasser gewaschen, das Waschwasser als schwerere Phase wird jeweils unten abgelassen. Anschließend wird die organische Phase unter Rühren und Vakuum destillativ vom Isobutanol befreit. Danach erhält man die klare, farblose, bei Raumtemperatur flüssige Formulierung von Tetraphenylphosphoniumphenolat und Phenol. Die Ausbeute nach der ³¹P-NMR-Methode beträgt 94 %.

Die Formulierung hat einen TPP-P-Gehalt von 42,1 Gew.% und einen Phenol-Gehalt von 57,8 Gew.%. Der Wassergehalt liegt unter 0,05 %, der Brom-Gehalt unter 0,01 % und der Natrium-Gehalt unter 0,5 ppm. Der Phosphor-Gehalt ist 3,3 %.

30 g der flüssigen Formulierung von Tetraphenylphosphoniumphenolat und Phenol nehmen ein Volumen von 47 ml ein.

Der Stockpunkt liegt bei -4°C.

### Beispiel 2

In einem 2-1-Rundkolben mit Rührer, Thermometer und Tropftrichter werden 225,6 g (2,40 mol) Phenol, 105 ml VE-Wasser und 83,86 g (0,2 mol) Tetraphenylphosphoniumbromid vorgelegt und bei 40°C gerührt. Innerhalb von ca. 5 Minuten werden 25,64 g (0,30 mol) 47 %ige Natronlauge zugetropft, der pH-Wert wird auf einen Bereich von 9,5 bis 11,0 eingestellt. Anschließend wird 1 h bei 50°C gerührt. Dann werden 160 g Isobutanol zugegeben. Nach der Phasentrennung wird die untere wässrige Phase abgelassen und die organische Phase fünfmal mit VE-Wasser gewaschen, das Waschwasser als schwerere Phase wird jeweils unten abgelassen. Anschließend wird die organische Phase unter Rühren und Vakuum destillativ vom Isobutanol befreit. Danach erhält man die klare, farblose, bei Raumtemperatur flüssige Formulierung von Tetraphenylphosphoniumphenolat und Phenol.

Die Formulierung hat einen TPP-P-Gehalt von 37,6 Gew.% und einen Phenol-Gehalt von 62,3 Gew.%. Der Wassergehalt liegt unter 0,05 %, der Brom-Gehalt unter 0,01 % und der Natrium-Gehalt unter 0,5 ppm. Der Phosphor-Gehalt ist 2,8 %. 30 g der flüssigen Formulierung von Tetraphenylphosphoniumphenolat und Phenol nehmen ein Volumen von 51 ml ein.

### Beispiel 3

In einem 2-1-Rundkolben mit Rührer, Thermometer und Tropftrichter werden 282 g (3,00 mol) Phenol, 105 ml VE-Wasser und 83,86 g (0,2 mol) Tetraphenylphosphoniumbromid vorgelegt und bei 40°C gerührt. Innerhalb von ca. 5 Minuten werden 25,64 g (0,30 mol) 47 %ige Natronlauge zugetropft, der pH-Wert wird auf einen Bereich von 9,5 bis 11,0 eingestellt. Anschließend wird 1 h bei 50°C gerührt. Dann werden 160 g Isobutanol zugegeben. Nach der Phasentrennung wird die untere wässrige Phase abgelassen und die organische Phase fünfmal mit VE-Wasser gewaschen, das Waschwasser als schwerere Phase wird jeweils unten abgelassen. Anschließend wird die organische Phase unter Rühren und Vakuum destillativ vom Isobutanol befreit. Danach erhält man die klare, farblose, bei Raumtemperatur flüssige Formulierung von Tetraphenylphosphoniumphenolat und Phenol.

Die Formulierung hat einen TPP-P-Gehalt von 28,3 Gew.% und einen Phenol-Gehalt von 71,6 Gew.%. Der Wassergehalt liegt unter 0,05 %, der Brom-Gehalt unter 0,01 % und der Natrium-Gehalt unter 0,5 ppm. Der Phosphor-Gehalt ist 2,2 %.

30 g der flüssigen Formulierung von Tetraphenylphosphoniumphenolat und Phenol nehmen ein Volumen von 48 ml ein.

Der Stockpunkt liegt bei -21°C.

### Verwendungsbeispiele

B1) In einem 500 ml Dreihalskolben mit Rührer, Innenthermometer und Vigreuxkolonne (30 cm, verspiegelt) mit Brücke werden 45,66 g (0,20 mol) Bisphenol A und 46,70 (0,22 mol) Diphenylcarbonat eingewogen. Die Apparatur wird durch Anlegen von Vakuum und Spülen mit Stickstoff (3 mal) vom Luftsauerstoff befreit und das Gemisch auf 190°C aufgeheizt. Jetzt werden 0,0082 g (4 x 10⁻³ mol-%) der flüssigen Formulierung von Tetraphenylphosphoniumphenolat (TPP-P) hergestellt gemäß Beispiel 1, bezogen auf Bisphenol A, zugegeben und das entstehende Phenol bei 100 mbar über 30 Minuten nach dem Aufschmelzen abdestilliert. Dann wird die Temperatur bis auf 235°C gesteigert und weitere 10 Minuten abdestilliert. Nun wird das Vakuum stufenweise bis auf 60 mbar verbessert und die Temperatur in 10 Minuten auf 300°C erhöht. In weiteren 10 Minuten wird das Vakuum auf 0,1 mbar eingestellt und 30 Minuten gerührt. Man erhält ein farbhelles lösungsmittelfreies Polycarbonat mit einer relativen Lösungsviskosität von 1,215 (Dichlormethan, 25°C, 5 g/l). Die Farbzahl des Polycarbonats ist 0,25.
B2): In einem 500 ml Dreihalskolben mit Rührer, Innenthermometer und Vigreuxkolonne (30 cm, verspiegelt) mit Brücke werden 45,66 g (0,20 mol) Bisphenol A und 46,70 (0,22 mol) Diphenylcarbonat eingewogen. Die Apparatur wird durch Anlegen von Vakuum und Spülen mit Stickstoff (3 mal) vom Luftsauerstoff befreit und das Gemisch auf 190°C aufgeheizt. Jetzt werden 0,0122 g (4 x 10⁻³ mol-%) der flüssigen Formulierung von Tetraphenylphosphoniumphenolat (TPP-P) hergestellt gemäß Beispiel 3, bezogen auf Bisphenol A, zugegeben und das entstehende Phenol bei 100 mbar über 30 Minuten nach dem Aufschmelzen abdestilliert. Dann wird die Temperatur bis auf 235°C gesteigert und weitere 10 Minuten abdestilliert. Nun wird das Vakuum stufenweise bis auf 60 mbar verbessert und die Temperatur in 10 Minuten auf 300°C erhöht. In weiteren 10 Minuten wird das Vakuum auf 0,1 mbar eingestellt und 30 Minuten gerührt. Man erhält ein farbhelles lösungsmittelfreies Polycarbonat mit einer relativen Lösungsviskosität von 1,197 (Dichlormethan, 25°C, 5 g/l). Die Farbzahl des Polycarbonats ist 0,24.

Die Beispiele belegen klar die überraschende Überlegenheit des erfindungsgemäßen Verfahrens zur Herstellung einer bei Raumtemperatur flüssigen Formulierung von Tetraphenylphosphoniumphenolat und Phenol, das deutlich einfacher als das in der Literatur beschriebene ist und im großtechnischen Maßstab durchgeführt werden kann. Die Verwendung der bei Raumtemperatur flüssigen Formulierung von Tetraphenylphosphoniumphenolat und Phenol zur Herstellung von Polycarbonaten führt zu Polycarbonaten, welche eine gute Farbzahl aufweisen.

## Patentansprüche

1. Verfahren zur Direktherstellung von bei Raumtemperatur flüssigen, hochreinen Formulierungen von Tetraphenylphosphoniumphenolat mit Phenol durch Umsetzung von Tetraphenylphosphoniumhalogeniden und Phenol in wässrig-alkalischer Lösung, **dadurch gekennzeichnet, dass** nach der Umsetzung Alkohole in Mengen von 50 Gew.% bis 200 Gew.% bezogen auf die Gewichtsmenge wässriger Reaktionslösung zugesetzt werden, wobei die Alkohole eine Löslichkeit in reinem Wasser von maximal 20 Gew.% haben.

2. Verfahren gemäß Anspruch 1, **dadurch gekennzeichnet, dass** die Umsetzung bei Temperaturen von 0 bis 55°C durchgeführt wird.

3. Verfahren gemäß mindestens einem der Anspruch 1 oder 2, **dadurch gekennzeichnet, dass** die Umsetzung bei Molverhältnissen von Phenol zu Phosphoniumhalogenid von ≥ 10 durchgeführt wird.

4. Verfahren gemäß mindestens einem der Ansprüche 1 bis 3, **dadurch gekennzeichnet, dass** die Umsetzung bei pH-Werten von 9.5 bis 11 durchgeführt wird.

5. Verfahren nach Anspruch 1 bis 4, **dadurch gekennzeichnet, dass** der verwendete Alkohol Isobutanol ist.

6. Phosphoniumphenolate erhältlich nach einem Verfahren wie in den Ansprüchen 1 bis 6 definiert.

7. Phosphoniumphenolate nach Anspruch 6 mit einem Halogenidgehalt von nicht mehr als 0.1 Gew.-%.

8. Phosphoniumphenolate nach Anspruch 6 mit einem Alkaligehalt von nicht mehr als 0,5 ppm.

9. Verwendung der in Anspruch 6 definierten Phosphoniumphenolate als Katalysator für Umesterungsreaktionen.

10. Verwendung der in Anspruch 6 definierten Phosphoniumphenolate als Umesterungskatalysator für die Polycarbonatherstellung.
